# EUROPEAN PATENT APPLICATION

(11) **EP 1 547 596 A1**
(43) Date of publication of application: **29.06.2005**
(21) Application number: 03794245.5
(22) Date of filing: 05.09.2003
(51) Int. Cl.: A61K 31/4409, A61K 33/24, A61P 35/00

(54) **MEDICINAL COMPOSITION AND METHOD FOR TREATING MALIGNANT TUMOR AND UTILIZATION THEREOF**

(30) Priority: 06.09.2002 JP 2002261226
(71) Applicant: D. Western Therapeutics Institute, Nagoya-shi, Aichi 466-0825 (JP); Nippon Shinyaku Co., Ltd., Kyoto-shi, Kyoto 601-8550 (JP)
(72) Inventor: HIDAKA, Hiroyoshi, Nagoya-shi, Aichi 468-0063 (JP); MATSUDA, Masato, Otsu-shi, Shiga 520-0225 (JP); KATOH, Fumitake, Takatsuki-shi, Osaka 569-0076 (JP)
(74) Representative: Hayes, Adrian Chetwynd
(86) International application number: PCT/JP2003/011338
(87) International publication number: WO 2004/022056

(57) **Abstract**

The present invention provides an excellent method and a pharmaceutical composition for treating a malignant tumor, and use thereof. Specifically, the present invention is related to a pharmaceutical composition for treating malignant tumor comprising a specific aminostilbazole derivative or a pharmaceutically acceptable salt thereof, which is administered in combination with chemotherapy and/or radiotherapy, a method of treatment and use of said compound.

## Description

### TECHNICAL FIELD

The present invention relates to a method for treating malignant tumor, a pharmaceutical composition and use thereof. More specifically, the present invention relates to a method for treating malignant tumor which comprises administering a specific aminostilbazole derivative or a pharmaceutically acceptable salt thereof in combination with chemotherapy and/or radiotherapy, a pharmaceutical composition therefor, and use thereof.

### BACKGROUND ART

Antitumor agents for treatment of malignant tumor can be classified into the following groups depending on the mechanism of action: alkylating agents, topoisomerase acting agents, antimetabolites, microtubule acting agents, antitumor antibiotics, plant alkaloids, hormones, enzymes and platinum compounds.

Generally, malignant tumors are treated by multiple drug therapy wherein many kinds of antitumor agents are administered in combination. The most significant merits of combined administration of antitumor agents exist in that it allows coping with diversity of an enormous number of tumor cells (about 10⁹ cells/cm³). That is, such enormous number of tumor cells include subgroups of cells being different in sensitivity or resistance to antitumor agents, and hence monotherapy with a single antitumor agent is relatively apt to raise the subgroup of cells resistant to said agent. Accordingly, a combined administration of plural tumor agents acting through different mechanisms of action would be useful to avoid development of drug resistance and also to attain better therapeutic results. In this sense, it is necessary to select a combination of antitumor agents of low cross resistance.

### DISCLOSURE OF INVENTION

The present inventors have intensively studied to obtain an antitumor agent with higher antitumor effect. The present inventors have found that, when a specific aminostilbazole derivative or a pharmaceutically acceptable salt thereof is used in combination with another antitumor agent(s) or radiotherapy, an excellent therapeutic effect can be achieved and accomplished the present invention.

Thus, the present invention provides the followings.
(1) A pharmaceutical composition for treating malignant tumor, which is administered in combination with another antitumor agent(s) and which comprises a compound of the formula (I) or a pharmaceutically acceptable salt thereof: 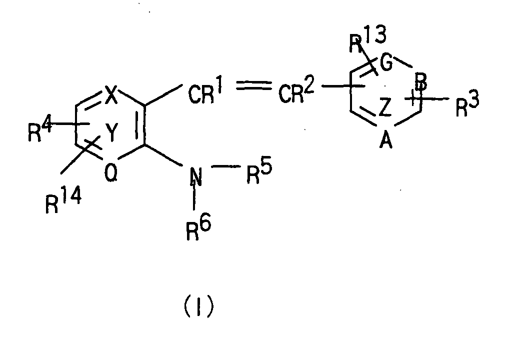 wherein R¹ and R² are the same or different and each represents hydrogen, alkyl of 1-6 carbon atoms, acyl of 1-6 carbon atoms, cyano, or -COOR (R represents hydrogen or alkyl of 1-6 carbon atoms);
   R³, R⁴, R¹³ and R¹⁴ are the same or different and each represents hydrogen, alkyl of 1-6 carbon atoms, alkoxy of 1-6 carbon atoms, halogenoalkoxy of 1-6 carbon atoms, acyl of 1-6 carbon atoms, acyloxy of 1-6 carbon atoms, hydroxy, halogen, nitro, cyano, amino, acylamino of 1-6 carbon atoms, aminoalkoxy of 1-6 carbon atoms, or morpholinoalkoxy with 1-6 carbon atoms in the alkyl moiety;
   R³ and R¹³ or R⁴ and R¹⁴ may independently combine together to form methylenedioxy;
   R⁵ represents (1) hydrogen, (2) alkyl of 1-6 carbon atoms which is optionally substituted by halogen, amino, monoalkylamino of 1-6 carbon atoms, dialkylamino of 1-6 carbon atoms, morpholino, alkoxy of 1-6 carbon atoms, or hydroxy, (3) alkenyl of 2-6 carbon atoms which is optionally substituted by halogen, (4) alkynyl of 2-6 carbon atoms, or (5) acyl of 1-6 carbon atoms;
   R⁶ represents (1) aroyl of 7-11 carbon atoms which is optionally substituted by alkyl of 1-6 carbon atoms, alkoxy of 1-6 carbon atoms, or halogen or (2) arylsulfonyl of 6-10 carbon atoms which is optionally substituted by alkyl of 1-6 carbon atoms, alkoxy of 1-6 carbon atoms, halogenoalkoxy of 1-6 carbon atoms, hydroxy, nitro, or halogen; and A, B, G, Q and X may be the same or different and each represents N, CH, N→O, or N⁺-(R⁷)E⁻ (R⁷ represents alkyl of 1-6 carbon atoms or arylalkyl of 7-14 carbon atoms; E⁻ represents a counterion for N⁺); provided that those wherein A, B, and G concurrently represent N, and those wherein A, B, G, Q, and X concurrently represent CH are excluded; and when any of A, B, G, Q and X represents N→O or N⁺-(R⁷)E⁻, only either of X or Q on Ring Y and/or only one of A, B and G on Ring Z can represent N→O or N⁺-(R⁷)E⁻. Also provided are a method of such treatment and use of the same.
   Preferably, the present invention provides a pharmaceutical composition which is used in combination with another antitumor agent(s) selected from platinum compounds, topoisomerase acting agents, microtubule acting agents and antitumor antibiotics, treatment with the same and use thereof.
   Specifically, the present invention provides a pharmaceutical composition, which composition is administered concurrently with or contains another antitumor agent(s), or which composition is administered together with another antitumor agent(s) in sequential manner, that is, the other antitumor agent is administered before or after the present composition, and such treatment and use thereof.
(2) A kit for carrying out the combined administration of a preparation comprising a compound of the formula (I) wherein R¹, R², R³, R⁴, R¹³, R¹⁴, R⁵, R⁶, A, B, G, Q and X are as defined above, or a pharmaceutically acceptable salt thereof with a preparation comprising another antitumor agent(s) for treatment of malignant tumor.
(3) A pharmaceutical composition for treating malignant tumor comprising a compound of the formula (I) wherein R¹, R², R³, R⁴, R¹³, R¹⁴, R⁵, R⁶, A, B, G, Q and X are as defined above, or a pharmaceutically acceptable salt thereof, which is administered in combination with radiotherapy for malignant tumor, a method of treatment, or use of said compound, specifically, a pharmaceutical composition that is administered concurrently with, before or after application of radiotherapy, a method of treatment, or use of the compound.

### BRIEF DESCRIPTION OF DRWAINGS

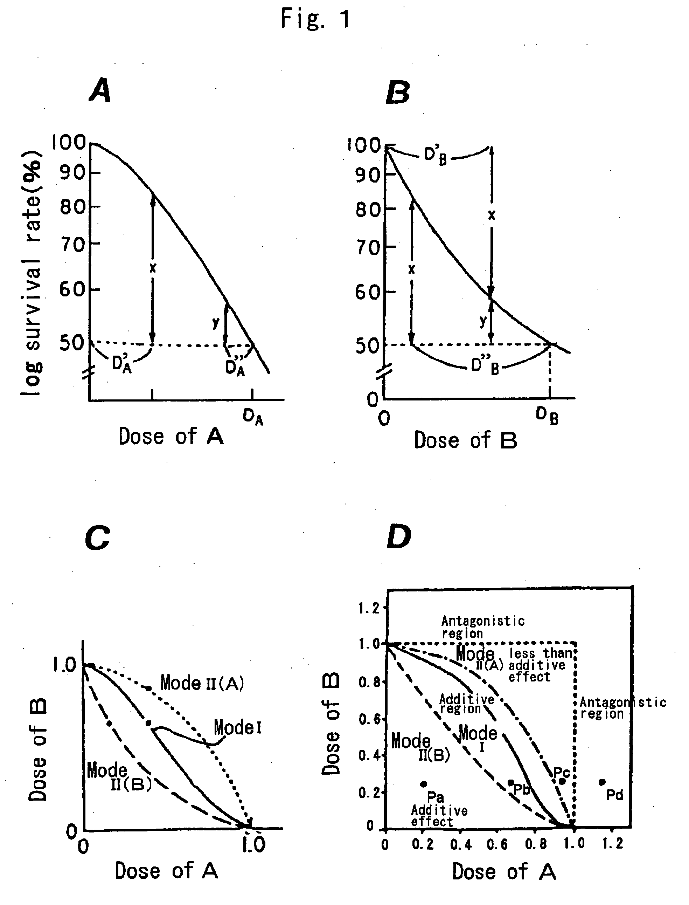

Fig. 1 shows the method of calculating the region wherein drugs A and B act additively upon combined administration, and the method of analyzing actual data.

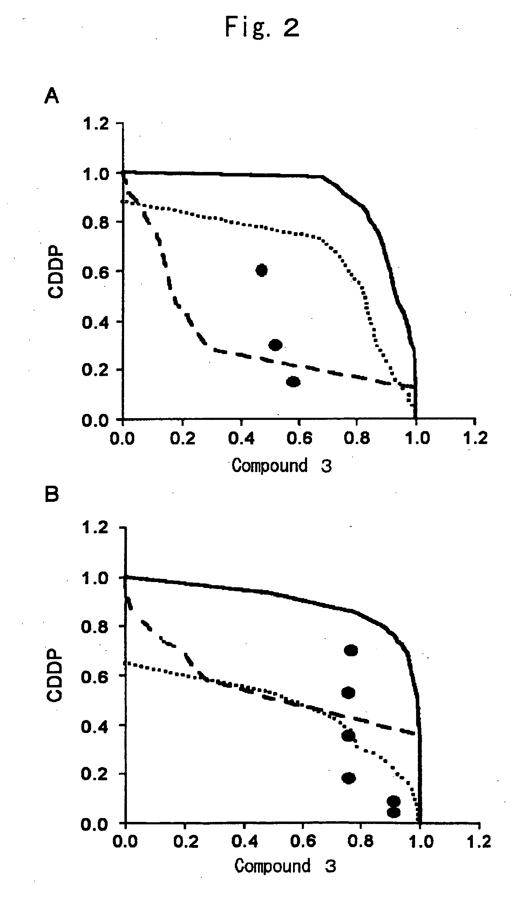

Fig. 2 shows additive or synergistic inhibitory effect on the cell growth when Compound 3 and CDDP are administered in combination. A shows the results where Compound 3 was administered first and B shows the results where CDDP was administered first. In the figure, the solid line (-), broken line (···) and dotted line (--) represent Mode I, Mode II: Compound 3 and Mode II: CDDP, respectively. The plots (•) in the figure correspond to the relative values between the 50% growth inhibitory concentration of Compound 3 and CDDP determined from the dose-response curve for combined administration at various combinations of concentration and the IC₅₀ value for Compound 3 and CDDP upon single administration.

### BEST MODER FOR CARRYING OUT THE INVENTION

### (1) Pharmaceutical Composition for Treating Malignant Tumor

The present invention will be hereinafter described in detail regarding the aminostilbazole derivative represented by the formula (I) which is essential for the pharmaceutical composition of the present invention.

In the definition of formula (I), the term "alkyl" means a straight-chain or branched-chain alkyl group of 1-6 carbon atoms, such as methyl, ethyl, n-propyl, isopropyl, n-butyl, isobutyl, sec-butyl, tert-butyl, n-pentyl, isopentyl, n-hexyl, isohexyl, etc. In particular, an alkyl of 1-3 carbon atoms is preferred.

The term "alkoxy" means a straight-chain or branched-chain alkoxy group of 1-6 carbon atoms, such as methoxy, ethoxy, n-propoxy, isopropoxy, n-butoxy, isobutoxy, sec-butoxy, tert-butoxy, n-pentyloxy, isopentyloxy, n-hexyloxy, isohexyloxy, etc. In particular, an alkoxy of 1-3 carbon atoms is preferred.

The term "alkenyl" means a straight-chain or branched-chain alkenyl group of 2-6 carbon atoms, such as ethenyl, 1-propenyl, 2-propenyl, isopropenyl, 2-butenyl, 3-butenyl, isobutenyl, methalyl, prenyl, isoprenyl, 1,1-dimethylallyl, etc. In particular, an alkenyl of 2-4 carbon atoms is preferred.

The term "alkynyl" means a straight-chain or branched-chain alkynyl group of 2-6 carbon atoms, such as ethynyl, 1-propynyl, 2-propynyl, 2-butynyl, 3-butynyl, 3-methyl-2-butynyl, etc. In particular, an alkynyl of 2-4 carbon atoms is preferred.

The term "acyl" means a straight-chain or branched-chain alkanoyl of 1-6 carbon atoms, such as formyl, acetyl, propionyl, butyryl, isobutyryl, valeryl, isovaleryl, pivaloyl, etc. The acyl may be substituted by halogen as is the case with trifluoroacetyl. In particular, an acyl of 2-4 carbon atoms is preferred.

The term "aroyl" means an aroyl of 7-11 carbon atoms, such as benzoyl, α-naphthoyl, β-naphthoyl, etc. Benzoyl is particularly preferred.

The term "aryl" of "arylsulfonyl" or "arylalkyl" includes an aryl of 6-10 carbon atoms, such as phenyl, α- naphthyl, β-naphthyl, etc. Phenyl is particularly preferred.

The term "halogen" includes chlorine, fluorine, bromine and iodine.

The term "halogenoalkyl" or "halogenoalkoxy" means an "alkyl" or "alkoxy" which is substituted by one or more halogens.

"Ring Y" which is defined by the groups: X and Q includes phenyl, pyridyl, and pyrazinyl. A substituted or unsubstituted phenyl is particularly preferred. In the case of substituted phenyl, the substituents R⁴ or R¹⁴ is preferably positioned at X and/or Q.

"Ring Z" which is defined by the groups: A, G and B includes phenyl, 2-pyridyl, 3-pyridyl, 4-pyridyl, pyrazinyl, 2-pyrimidinyl, 4-pyrimidinyl, 5-pyrimidinyl, 3-pyridazinyl, 4-pyridazinyl, and N-oxide thereof. Above all, pyridyl is preferred, and an optionally substituted 4-pyridyl or N-oxide thereof is particularly preferred. In the case of substituted 4-pyridyl or N-oxide thereof, either of the substituents R³ and R¹³ is preferably attached at 3-position.

The "counterion for N⁺" of "E⁻" in the definition of N⁺-(R⁷)E⁻ means an anion such as halogen ion, chlorate ion, and nitrate ion.

Preferred compounds to be used in the present invention are those of the formula (I) wherein R¹ and R² are both hydrogen; R³, R⁴, R¹³ and R¹⁴ are the same or different and each represents hydrogen, acyl of 2-4 carbon atoms, halogen or hydroxy; R⁵ represents hydrogen, alkyl of 1-3 carbon atoms substituted by hydroxy, or acyl of 2-4 carbon atoms; R⁶ represents phenylsulfonyl substituted by alkoxy of 1-3 carbon atoms; Ring Y is phenyl; and Ring Z is 4-pyridyl or N-oxide thereof, or a pharmaceutically acceptable salt thereof.

More preferred compounds are those of the formula (I), wherein R¹ and R² are both hydrogen; R³, R⁴, R¹³ and R¹⁴ are the same or different and each represents hydrogen, acetyl, fluorine or hydroxy; R⁵ represents hydrogen, ethyl substituted by hydroxy, or acetyl; R⁶ represents phenylsulfonyl substituted by methoxy; Ring Y is phenyl; and Ring Z is 4-pyridyl or N-oxide thereof, or a pharmaceutically acceptable salt thereof.

Still further preferred compounds are those of the formula (I) wherein R¹ and R² are hydrogen, -NR⁵R⁶ is (p-methoxyphenyl)sulfonylamino, N-acetyl-N-[(p-methoxyphenyl)-sulfonyl]amino or N-(hydroxyethyl)-N-[(p-methoxyphenyl)-sulfonyl]amino, R³ and R¹³ are the same or different and each represents hydrogen, hydroxy, acetyloxy or fluorine, Ring Z is 4-pyridyl or N-oxide thereof, R⁴ and R¹⁴ are the same or different and each represents hydrogen, hydroxy or methoxy, and Ring Y is phenyl.

The compounds usable in the present invention can be prepared by, for example, a method described in WO95/27699.

Examples of a pharmaceutically acceptable salt of compound (I) usable in the present invention include salts with inorganic acids such as hydrochloric acid, sulfuric acid, nitric acid, phosphoric acid, hydrofluoric acid, hydrobromic acid, etc., and salts with organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid, etc. When R¹ or R² is COOH, examples of salts include a salt with alkali metals or alkaline earth metals such as sodium, potassium, calcium, etc. These salts of compound (I) can be prepared in a known process.

Especially preferred compounds to be used in the present invention are listed below:
(E)-4-[2-[2-[N-[(p-methoxyphenyl)sulfonyl]amino]-phenyl]ethenyl]pyridine (Compound 1) and hydrochloride thereof,
(E)-4-[2-[2-[N-acetyl-N-[(p-methoxyphenyl)sulfonyl]-amino]phenyl]ethenyl]pyridine 1-oxide (Compound 2)
(E)-4-[2-[2-[N-[(p-methoxyphenyl)sulfonyl]amino]-phenyl]ethenyl]pyridine 1-oxide (Compound 3),
(E)-4-[2-[2-[N-acetyl-N-[(p-methoxyphenyl)sulfonyl]-amino]phenyl]ethenyl]pyridine (Compound 4),
(E)-4-[2-[2-[N-(2-hydroxyethyl)-N-[(p-methoxyphenyl)-sulfonyl]amino]phenyl]ethenyl]pyridine 1-oxide (Compound 5), and
(E)-4-[2-[2-[N-(2-hydroxyethyl)-N-[(p-methoxyphenyl)-sulfonyl]amino]phenyl]ethenyl]pyridine (Compound 6).

Above all, Compounds 1, 2 and 3 are preferred.

The present applicants have found aminostilbazole derivatives with anticancer activity, and made a patent application and obtained a patent (WO95/27699, Japanese Patent No. 3080405). JP 3080405 discloses that the aminostilbazole derivative as claimed herein has an excellent anticancer activity with low toxicity and is orally administrable, whereby it can be a therapeutic agent for malignant tumor of various types. However, JP 3080405 does not provide any detailed descriptions regarding the mechanism of action.

The compound (I) has proved to inhibit cell division by acting on a site other than microtubule, and thereby inducing apoptosis specifically during M phase. Further, inhibition of activity of apoptosis-related proteins such as p53, p21 or caspase hardly affected said beneficial effects. The mechanism of resistance is unknown due to lack of cells with especially low sensitivity or resistance. This fact would itself indicate the low incidence of resistant cells. In addition, research on metabolism revealed that many portions are excreted without change, which means that the compound (I) is unsusceptible to the detoxification mechanism involving glutathione such as GST or GSH. In fact, the compound (I) did not show cross resistance to cell lines resistant to typical antitumor agents such as Adriamycin®, cisplatin or Taxol®, and exhibited antitumor action effectively.

In this sense, the pharmaceutical composition of the present invention comprising a compound of the formula (I) or a pharmaceutically acceptable salt thereof is useful in treatment of a patient suffering from a malignant tumor resistant to typical antitumor agents.

There are no limitations regarding the other antitumor agent(s) to be used in the present invention. However, considering the above, the following antitumor agents are examplified, which agents are expected to cause cross resistance in extremely low frequency.

### 1. Platinum compounds, for example, cisplatin (CDDP), carboplatin

The site of action of platinum compounds is the crosslinking between the strands of double-stranded DNA. The mechanism of development of resistance is related to the enhanced expression of a protein functioning in the repair of DNA damaged by a platinum compound such as HMG1, etc.; efflux by multidrug-resistant protein (MRP); metabolic detoxification by metabolic enzyme system (glutathione transferase and metallothionein); or decrease of apoptosis-inducing mechanism due to nucleic acid mutation caused by p53 or p21, etc.

### 2. Topoisomerase acting agents, for example, camptothecin, irinotecan, topotecan, etoposide, etc.

The site of action is DNA topoisomerase, and these agents break DNA through inhibition of DNA reunion activity of the enzyme. The mechanism of development of resistance is related to the efflux by multidrug-resistant protein MDR/MRP; structural or quantitative change of target enzyme (TopoI or TopoII); or decrease of apoptosis-inducing mechanism due to nucleic acid mutation caused by p53 or p21, etc.

### 3. Microtubule acting agents, for example, Vincristine®, Vinblastine®, Navelbine®, Taxol®, Taxotere®, etc.

The site of action is microtubule. These agents bind to microtubule and thereby inhibiting tubulin polymerization or depolimerization. The mechanism of development of resistance is related to the efflux by multidrug-resistant protein MDR/MRP; or decrease of target-binding ability due to mutation of tubulin, a protein composing microtubule, or change of subtype.

### 4. Antitumor antibiotics, for example, doxorubicin (Adriamycin®), bleomycin, cyclophosphamide, anthracycline, etc.

These agents generally act on plural sites. The mechanism of action is not necessarily clear; however, it possibly is damage of DNA by a radical (doxorubicin, bleomycin), topoisomerase (doxorubicin), inhibition of RNA synthesis (cyclophosphamide), etc. The mechanism of development of resistance is related to glutathione-dependent detoxification (GSH, GST); or the efflux by multidrug-resistant protein MDR/MRP.

Specifically, the pharmaceutical composition of the present invention is administered with another antitumor agent(s) simultaneously or sequentially.

Simultaneous administration can be carried out by administering a compound (I) or a pharmaceutically acceptable salt and another antitumor agent(s) in discrete preparations or in one preparation.

"Sequential administration" means that a compound (I)

or a pharmaceutically acceptable salt and another antitumor agent are alternatively administered with a given time interval. For the present invention, the compound (I) of the present invention may be administered before or after another antitumor agent. The "given time interval" is generally determined depending on the pharmacodynamics of the firstly administered antitumor agent. For example, in case that a compound (I) is administered first, the time interval will be determined taking the progress of cell cycle into consideration, as from the time when the concentration decreased to such an extent that the cell cycle arrest activity disappears.

The pharmaceutical composition of the present invention is used in combination with a therapeutically effective amount of another antitumor agent(s). In general, it is prepared by mixing a compound of the formula (I) or a pharmaceutically acceptable salt thereof in association with a pharmaceutically acceptable carrier or excipient therefor. The present pharmaceutical composition can be administered orally or parenterally.

For oral administration, the present composition can be in the form of tablet, pill, granule, powder, capsule, syrup, emulsion, suspension, and the like. Such formulations can be prepared in a known process using a carrier or excipient such as lactose, starch, sucrose, magnesium stearate, and the like. For parenteral administration, the present composition can be in the form of injection, suppository, external preparation, and the like. The injections may be adapted to intravenous, subcutaneous or intramuscular injection, drip infusion, or the like. The injection is generally provided after filling in an appropriate ampoule. Examples of suppository include rectal and vaginal suppositories. Examples of external preparation include ointment (including cream) and preparations for nasal or transdermal administration.

The content, namely, the therapeutically effective amount, of a compound (I) or a pharmaceutically acceptable salt thereof in the preparations above can be selected from the range of 0.1-99.5%, preferably 0.5%-90%. The dosage per body surface area varies depending on age, sexuality, severity of disease, dosage of another antitumor agent to be combined; however, it would be selected from 0.1-1000mg/m², preferably, 1-100mg/m², in the case of oral and intravenous administrations. The present pharmaceutical composition as defined above is administered together with a therapeutically effective amount of another antitumor agent.

The present pharmaceutical composition is useful in treatment of malignant tumor. Examples of such malignant tumor include lung cancer, breast cancer, gastrointestinal cancer, prostate cancer, blood cancer, etc., but are not limited thereto.

### (2) Kit for Carrying out the Combined Administration for Treatment of Malignant Tumor

In another embodiment, the present invention provides a kit for carrying out the combined administration for treatment of malignant tumor.

As mentioned above, the pharmaceutical composition of the present invention is prepared by formulating a pharmaceutically acceptable carrier or an excipient and administered orally or parenterally. The resultant preparation can be used to form a kit together with another preparation separately prepared using a different antitumor agent. When the preparations are powders for injection, the kit may include a diluent or the like to be used for obtaining an injection before use. With the diluent for preparing injection before use, the compound (I) and another antitumor agent can be administered as a single injection. Further, the kit of the present invention may contain preparations formulated separately in discrete forms so that one can administer the compound (I) and another antitumor agent to a subject through the same or different routs simultaneously or sequentially with a time interval.

### (3) Pharmaceutical Composition for Treatment of Malignant Tumor Administered in Combination with Radiation Therapy

In further embodiment, the present invention provides a pharmaceutical composition which is administered in combination with not only the other antitumor agent, but also radiotherapy, a method of treatment and the use of the compound (I).

The site of action of the radiotherapy is mechanical or chemical damage in DNA, wherein the chemical damage is caused by liberated radical. There are no specific points in the cell cycle. The mechanism of resistance of radiotherapy is activation of DNA repairing system, reduction of cell death inducing mechanism due to nucleic acid mutation caused by p53 or p21, etc., and enhancement of radical digestive function such as SOD etc. Accordingly, this embodiment would also be a combination of relatively low in development of cross resistance.

Specifically, the pharmaceutical composition of the present invention is generally administered before conducting radiotherapy; however, it can be administered simultaneously with or after the radiotherapy. For example, the pharmaceutical composition of the present invention can be administered once a day in the morning for continuous 5 days, and the radiotherapy can be conducted in the afternoon of day 5.

The present invention is explained in more detail by way of examples below, which however should not be construed as limiting the scope of the invention.

### Example 1

### Effect of Combined Administration of (E)-4-[2-[2-[N-[(p-methoxyphenyl)sulfonyl]amino]phenyl]ethenyl]pyridine 1-oxide (Compound 3) and Cisplatin (in vitro)

Effect of combined administration of Compound 3 and cisplatin in simultaneous or sequential manner was examined in vitro.

Compound 3 was prepared in a manner similar to that described in WO95/27699. Cisplatin (CDDP; cis-platinum (II) diamine chloride) was purchased from Sigma. The effect of combination therapy was analyzed by means of isobologram which has been established by Steel and Peckham and is commonly used in vitro for evaluating effect of combined administration of antitumor agents. G. Gordon Steel and Michael J. Peckham, Int. J. Radiation Oncology Biol. Phys., 5: 85-91, 1979; T. Okano, T.Ohnuma, James F. Holland, H. Phillip Koeffler and Han Jui, Investigational New Drugs, 1: 145-150, 1983.

### 1. Brief Description of Isobologram Analysis

Isobologram analysis for evaluating the effect of combined administration of drugs will hereinafter be described briefly making reference to Fig. 1. The detailed method is described in the above-mentioned literature or "Gan-no-Kenkyu, 5. Sensitivity Test, 2. Prediction of Combination Therapy, Dobunshoin".

### 1-1 Preparation of Isobologram

The combined administration of antitumor agents A and B can be determined in the following manner. A dose-response curve for each of antitumor agents A and B in single administration is obtained, and therefrom is determined IC₅₀ (50% cell growth inhibitory concentration) of each agent. Fig. 1A and 1B.

DA, D'A, D''A, DB, D'B and D''B are obtained from the dose-response curve in Fig. 1A and 1B, and used to construct curves for Mode I, Mode II (A) and Mode II(B) in Isobologram (Fig. 1C). Mode I curve is derived from D'A and D'B. Mode II(A) is derived from D'A and D''B, and Mode II(B) from D'B and D''A.

### I-2 Determination of Effect of Combined Administration by Isobologram

Fig. 1D shows data points Pa, Pb, Pc and Pd plotted on Fig. 1C for the sake of convenience, said data points having been obtained in combined administration.

The effect is determined in the following manner.

When a data point enters in the region surrounded by Mode I and Mode II (additive region (envelope of additivity) in Fig. 1D, Pb), that is, the dose required for IC₅₀ in the combined administration is equivalent to the predictive dose, the two antitumor agents show additive effect. When a data point enters in the left lower region (Pa), that is, the dose in the combined administration is less than the predictive one, the two antitumor agents show synergistic action.

When a data point enters in the right upper region, that is, the dose in the combined administration is more than the predictive one, the effect is determined to be less than additive effect (Pc) and within antagonistic region (protection, Pd). The data point Pc is within the square, which means that the combined administration has an advantage; however, only additive effect is attained. The data point Pd is outside of the square, which means that single use has advantage over the combined administration.

### 2. Experimental Method

### 2-1 Preparation of Dose-Response Curve in Single Administration of Compound 3 and Cisplatin

Compound 3 and cisplatin (CDDP) each was dissolved in dimethylsulfoxide (DMSO: Nacalai Tesque, Inc.). As to the solution of Compound 3, an arithmetic dilution series was prepared in 10% FBS-supplemented DMEM medium (Nissui Pharmaceutical, Co., Ltd.) from 0.22 µg/m to 0.01 µg/ml with an increment of 0.01 µg/ml. As to the solution of CDDP, an arithmetic dilution series were prepared at concentrations of 50.0, 40.0, 30.0, 20.0, 10.0, 9.0, 8.0, 7.0, 6.0, 5.0, 4.0, 3.0, 2.7, 2.4, 2.1, 1.8, 1.5, 1.2, 0.9, 0.6, 0.3, 0.2 and 0.1 µg/ml. WiDr human colon adenocarcinoma cell lines (American Type Culture Collection, Manassas, VA 20108 USA) were grown in 96 well plate at 37°C, 5% CO₂ for 1 or 2 days. To the culture (100 µl, 3×10⁴ cells/ml) was added the dilution (100 µl) of each drug prepared above. After 24 hours, the medium was removed from the plate, and the well was washed twice with PBS (Nissui Pharmaceutical, Co., Ltd.). Medium (200 µl) was added to the culture followed by incubation for another 4 days. After cultivation, a dose-response curve for both agents in the single administration was prepared by MTT assay, and IC₅₀ value was determined.

### 2-2 Determination of IC₅₀ in Combined Administration of Compound 3 and CDDP

A 2-fold delusion series starting from 0.3 µg/ml (8 steps in total) of Compound 3 was prepared. WiDr human colon adenocarcinoma cell lines were grown for 1 day. To the medium was added serial dilution (50 µl) of Compound 3 and 10% FBS-supplemented DMEM medium (150 µl), and the culture grown for 24 hours. The resultant growth medium (100 µl) and a solution (100 µl) containing CDDP at 2-fold concentration of the intended concentration (4.0, 3.0, 2.0, 1.0, 0.5, 0.25, 0.125) were combined and cultured for 24 hours. A dose-response curve for every combination of concentrations was prepared by MTT assay. The same experiment was conducted by exchanging Compound 3 and CDDP. From the curves obtained in these experiments, a combination of concentrations exerting 50% growth inhibitory activity was obtained.

### 3. Results and Discussion

Fig. 2 shows effect of combined administration of Compound 3 and CDDP by Isobologram of IC₅₀. Fig. 2A shows the effect of combined administration wherein 24-hour-treatment with Compound 3 is followed by 48-hour-treatment with CDDP. Fig. 2B shows the effect of combined administration wherein 24-hour-treatment with CDDP is followed by 48-hour-treatment with Compound 3.

Fig. 2A and 2B show that the combined administration of Compound 3 and CDDP provides additive or synergistic inhibitory effect irrespective of the order of administration thereof.

It was revealed that a combined administration of Compound 3 and CDDP exerts potent inhibitory activity on the cell growth than that a single administration of respective compounds exerts.

### Example 2

Antitumor Effect of (E) -4- [2- [2- [N-acetyl-N- [(p-methoxyphenyl)sulfonyl]amino]phenyl]ethenyl]pyridine 1-oxide (Compound 2) on Murine Monocytic Leukemia P388 Transplanted into Mouse (Combination Effect of Compound 2 and Cisplatin)

### 1. Tumor for Transplantation

Monocytic leukemia cell line P388 was purchased from Japanese Foundation for Cancer Research, Cancer Chemotherapy Center. The cryopreserved tumor was transplanted intraperitoneally into DBA/2 mouse (Japan SLC, Inc.). When the abdominal bloating was observed, ascites was removed and subjected to the test.

### 2. Test Drug and Preparation Thereof

1) Test Drug
   Compound 2 was prepared in accordance with the method described in WO95/27699.
2) Control Drug
   Cisplatin (CDDP) was purchased from Sigma.
3) Preparation of Pharmacutical Composition
   Compound 2 was weighed and ground down in an agate motor. It was then suspended slowly in the motor by adding 0.5% aqueous methylcellulose solution (M.C.) step by step to obtain 10 mg/ml suspension. The suspension was further diluted with M.C. to obtain a normal concentration and stored at 4 °C. CDDP was dissolved and diluted with physiological saline. Immediately after preparation, the CDDP solution was subjected to test. It is assured that the suspension of Compound 2 in M.C. is homogeneous and stable for 14 days in a cold place.

### 3. Animals and Conditions for Breeding

Male 4-week-old DBA/2 mice (n=10, 2 mice for the present test) and male 4-week-old CDF₁ mice (DBA/2 × BALB/c) (n=156) were purchased from Japan SLC, Inc., and used six animals per group. Animals were kept in a chamber maintained at room temperature of 21 to 25°C and humidity of 45-65% with a given lighting time intervals (light period: 7:00 to 19:00; dark period: 19:00 to 7:00) and allowed to access freely to feed (F-2, Funabashi Nojo) and tap water. The animals purchased were used in the test after subjecting to taming and quarantine for 1 week.

### 4. Test Method

The cryopreserved P388 cell lines were transplanted intraperitoneally into DBA/2 mouse. When the abdominal bloating was observed, ascites containing cancer cells were removed. The ascites was diluted with phosphate-buffered physiological saline (PBS, pH7.4) until the cell concentration became 5×10⁶ cells/ml. The resultant solution (0.2 ml) was transplanted intraperitoneally into CDF₁ mouse (1×10⁶ cells/mouse). The drug was administered to mouse (0.1 ml/10g body weight) on and after the next day of transplantation.

### 5. Administration Route of Drugs

Compound 2 was administered orally and CDDP intraperitoneally. Animals of control group were untreated because of diversity of administration route and complexity of administration schedule.

### 6. Administration Schedule

The administration was started on the next day (day 1) of transplantation of tumor according to the protocol shown in Table 1, i.e., day 1; day 2; or day 1 and 2.

### 7. Calculation of Survival Rate

In accordance with the screening protocol of National Cancer Institute (NCI, USA), survival rate T/C (%) was calculated from the median survival time (MST) of treated group (T), which has received a test drug, and control group (C), which has not received the drug.

### 8. Results and Discussion

Compound 2 was examined for the antitumor effect against mouse monocytic leukemia P388 transplanted into mouse, in combination with CDDP. The results are shown in Table 1.

**Table 1**

| Antitumor activity of Compound 2 in combination with CDDP on P388 murine monocyte leukemia transplanted into mice | | | | | | | | |
|---|---|---|---|---|---|---|---|---|
| | Day1 | | | Day2 | | | | Survival |
| Treatment | Dose (mg/kg) | Route | Treatment | Dose (mg/kg) | Route | MST^{a)} | T/C(%) | number on day50 |
| Control | | | | | | 10 | - | 0 |
| Compound 2 | 25 | p.o | - | | | 12.5 | 125 | 0 |
| Compound 2 | 50 | p.o | - | | | 13 | 130 | 0 |
| Compound 2 | 100 | p.o | - | | | 16.5 | 165 | 0 |
| - | | | Compound 2 | 25 | p.o | 13 | 130 | 0 |
| - | | | Compound 2 | 50 | p.o | 13 | 130 | 0 |
| - | | | Compound 2 | 100 | p.o | 15 | 150 | 0 |
| CDDP | 2.5 | i.p | - | | | 14.5 | 145 | 0 |
| CDDP | 5 | i.p | - | | | 17 | 170 | 0 |
| CDDP | 10 | i.p | - | | | 17 | 170 | 0 |
| - | | | CDDP | 2.5 | i.p | 15.5 | 155 | 0 |
| - | | | CDDP | 5 | i.p | 17 | 170 | 0 |
| - | | | CDDP | 10 | i.p | 17 | 170 | 0 |
| Compound 2 +CDDP | 25 2.5 | p.o i.p | - | | | 16 | 160 | 0 |
| Compound 2 +CDDP | 50 5 | p.o i.p | - | | | 24 | 240 | 0 |
| Compound 2 +CDDP | 100 10 | p.o i.p | - | | | 29 | 290 | 2 |
| Compound 2 | 25 | p.o | CDDP | 2.5 | i.p | 17.5 | 175 | 0 |
| Compound 2 | 50 | p.o | CDDP | 5 | i.p | 19.5 | 195 | 0 |
| Compound 2 | 100 | p.o | CDDP | 10 | i.p | 27 | 270 | 2 |
| CDDP | 2.5 | i.p | Compound 2 | 25 | p.o | 17 | 170 | 0 |
| CDDP | 5 | i.p | Compound 2 | 50 | p.o | 21.5 | 215 | 1 |
| CDDP | 10 | i.p | Compound 2 | 100 | p.o | >50 | >500 | 5 |
| N=6; ^{a)}MST, median survival time | | | | | | | | |

A test drug is effective when T/C value is 125% or higher according to the criterion for the screening protocol of NCI. When Compound 2 and CDDP are administered alone, T/C value is 125% or higher on day 1 or 2 as can be seen from Table 1. However, the survival rate on day 5 was not observed for either drug. When these drugs are administered in combination, the combined administration group shows higher T/C (%) value than the single administration group for every administration schedule. The combined administration wherein CDDP (10mm/kg) is administered on day 1 and Compound 2 (100mg/kg) on day 2 gave a survival rate of 5/6 even on day 50.

Clinically, an antitumor agent is not used alone. Rather, it is used for treatment in combination with another tumor agent acting through different mechanism of action so as to increase the antitumor effect and reduce the toxicity. The present experiment revealed that Compound 2 shows higher T/C (%) value when administed in combination with CDDP compared to single administration, indicating that the present composition would be clinically effective.

### FORMULATION EXAMPLES

| Formulation Example 1 (Hard capsules) | |
|---|---|
| In 220 mg per capsule: | |
| Compound 2 | 10 mg |
| Lactose | 187 mg |
| Microcrystalline cellulose | 20 mg |
| Magnesium stearate | 3 mg |

The above components were evenly mixed and using a capsule-filling machine, the mixture was filled in No. 2 capsule shells to provide hard capsules.

| Formulation Example 2 (Granules) | |
|---|---|
| In 1 g of granules: | |
| Compound 1 | 10 mg |
| Lactose | 880 mg |
| Low-substitution-degree | |
| hydroxypropylcellulose | 70 mg |
| Hydroxypropylcellulose | 40 mg |

The above components were evenly mixed followed by kneading, and using a granulator, the kneaded mass was granulated to a diameter of 0.7 mm to provide granules.

| Formulation Example 3 (Hard capsules) | |
|---|---|
| In 220 mg per capsule: | |
| Compound 2 | 10 mg |
| Cisplatin | 1 mg |
| Lactose | 186 mg |
| Microcrystalline cellulose | 20 mg |
| Magnesium stearate | 3 mg |

The above components were evenly mixed and using a capsule-filling machine, the mixture (220 mg) was filled in No. 2 capsule shells to provide hard capsules.

| Formulation Example 4 (Granules) | |
|---|---|
| In 1 g of granules: | |
| Compound 3 | 10 mg |
| Cisplatin | 1 mg |
| Lactose | 879 mg |
| Low-substitution-degree | |
| hydroxypropylcellulose | 70 mg |
| Hydroxypropylcellulose | 40 mg |

The above components were evenly mixed followed by kneading, and using a granulator, the kneaded mass was granulated to a diameter of 0.7 mm to provide granules.

### INDUSTRIAL APPLICABILITY

The present invention is related to a pharmaceutical composition for treating malignant tumor, which comprises a particular aminostilbazole derivative or a pharmaceutically acceptable salt thereof and which is administered in combination with chemotherapy and/or radiotherapy, a method for treatment, and use of said compound. In this manner, the present invention achieves higher antitumor effect than that attained by single administration of respective agents. Accordingly, the present invention is expected to be clinically useful by combined administration with a commercially available antitumor agent.

## Claims

1. A pharmaceutical composition for treating malignant tumor, which is administered in combination with another antitumor agent(s) and which comprises a compound of the formula (I) or a pharmaceutically acceptable salt thereof: 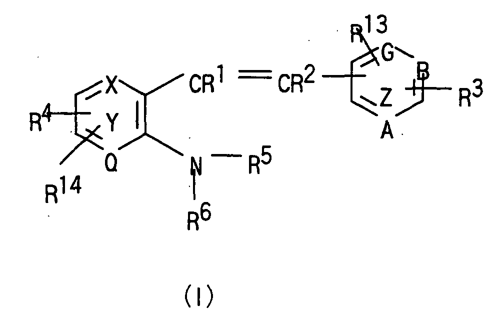 wherein R¹ and R² are the same or different and each represents hydrogen, alkyl of 1-6 carbon atoms, acyl of 1-6 carbon atoms, cyano, or -COOR (R represents hydrogen or Cl-6 alkyl);
R³, R⁴, R¹³ and R¹⁴ are the same or different and each represents hydrogen, alkyl of 1-6 carbon atoms, alkoxy of 1-6 carbon atoms, halogenoalkoxy of 1-6 carbon atoms, acyl of 1-6 carbon atoms, acyloxy of 1-6 carbon atoms, hydroxy, halogen, nitro, cyano, amino, acylamino of 1-6 carbon atoms, aminoalkoxy of 1-6 carbon atoms, or morpholinoalkoxy with 1-6 carbon atoms in the alkyl moiety;
R³ and R¹³ or R⁴ and R¹⁴ may independently combine together to form methylenedioxy;
R⁵ represents (1) hydrogen, (2) alkyl of 1-6 carbon atoms which is optionally substituted by halogen, amino, monoalkylamino of 1-6 carbon atoms, dialkylamino of 1-6 carbon atoms, morpholino, alkoxy of 1-6 carbon atoms, or hydroxy, (3) alkenyl of 2-6 carbon atoms which is optionally substituted by halogen, (4) alkynyl of 2-6 carbon atoms, or (5) acyl of 1-6 carbon atoms;
R⁶ represents (1) aroyl of 7-11 carbon atoms which is optionally substituted by alkyl of 1-6 carbon atoms, alkoxy of 1-6 carbon atoms, or halogen or (2) arylsulfonyl of 6-10 carbon atoms which is optionally substituted by alkyl of 1-6 carbon atoms, alkoxy of 1-6 carbon atoms, halogenoalkoxy of 1-6 carbon atoms, hydroxy, nitro, or halogen; and A, B, G, Q and X may be the same or different and each represents N, CH, N→O, or N⁺-(R⁷)E⁻ (R⁷ represents alkyl of 1-6 carbon atoms or arylalkyl of 7-14 carbon atoms; E⁻ represents a counterion for N⁺);
provided that those wherein A, B, and G concurrently represent N, and those wherein A, B, G, Q, and X concurrently represent CH are excluded; and when any of A, B, G, Q and X represents N→O or N⁺-(R⁷)E⁻, only either of X or Q on Ring Y and/or only one of A, B and G on Ring Z can represent N→O or N⁺-(R⁷)E⁻.

2. The pharmaceutical composition of claim 1, which comprises the compound of the formula (I) wherein R¹ and R² each represents hydrogen; R³, R⁴, R¹³ and R¹⁴ are the same or different and each represents hydrogen, acyl of 2-4 carbon atoms, halogen or hydroxy; R⁵ represents hydrogen, alkyl of 1-3 carbon atoms substituted by hydroxy or acyl of 2-4 carbon atoms; R⁶ represents phenylsulfonyl substituted by alkoxy of 1-3 carbon atoms; Ring Y is phenyl and Ring Z is 4-pyridyl or N-oxide thereof, or a pharmaceutically acceptable salt thereof.

3. The pharmaceutical composition of claim 2, which comprises the compound of the formula (I) wherein R¹ and R² each represents hydrogen; R³, R⁴, R¹³ and R¹⁴ are the same or different and each represents hydrogen, acetyl, fluorine or hydroxy; R⁵ represents hydrogen, ethyl substituted by hydroxy or acetyl; R⁶ represents phenylsulfonyl substituted by methoxy; Ring Y is phenyl and Ring Z is 4-pyridyl or N-oxide thereof, or a pharmaceutically acceptable salt thereof.

4. The pharmaceutical composition of claim 3, wherein the compound of the formula (I) is a compound selected from the group consisting of:
(E)-4-[2-[2-[N-[(p-methoxyphenyl)sulfonyl]amino]-phenyl]ethenyl]pyridine,
(E)-4-[2-[2-[N-[(p-methoxyphenyl)sulfonyl]amino]-phenyl]ethenyl]pyridine 1-oxide,
(E)-4-[2-[2-[N-(2-hydroxyethyl)-N-[(p-methoxyphenyl)-sulfonyl]amino]phenyl]ethenyl]pyridine 1-oxide,
(E)-4-[2-[2-[N-(2-hydroxyethyl)-N-[(p-methoxyphenyl)-sulfonyl]amino]phenyl]ethenyl]pyridine,
(E)-4-[2-[2-[N-acetyl-N-[(p-methoxyphenyl)sulfonyl]-amino]phenyl]ethenyl]pyridine 1-oxide, and
(E)-4-[2-[2-[N-acetyl-N-[(p-methoxyphenyl)sulfonyl]-amino]phenyl]ethenyl]pyridine, or a pharmaceutically acceptable salt thereof.

5. The pharmaceutical composition of any one of claims 1 to 4, wherein the other antitumor agent is selected from the group consisting of platinum compounds, topoisomerase acting agents, microtubule acting agents and antitumor antibiotics.

6. The pharmaceutical composition of any one of claims 1 to 5, which is administered simultaneously with another antitumor agent.

7. The pharmaceutical composition of any one of claims 1 to 5, which further contains another antitumor agent.

8. The pharmaceutical composition of any one of claims 1 to 5, wherein the other antitumor agent is administered sequentially.

9. A kit for combined administration for the treatment of malignant tumor, which comprises a preparation containing a compound of the formula (I) wherein R¹, R², R³, R⁴, R¹³, R¹⁴, R⁵, R⁶, A, B, G, Q and X are the same as defined above, or a pharmaceutically acceptable salt thereof, and a preparation comprising another antitumor agent.

10. A pharmaceutical composition for treating malignant tumor, which is administered in combination with radiotherapy for malignant tumor and which comprises a compound of the formula (I) wherein R¹, R², R³, R⁴, R¹³, R¹⁴, R⁵, R⁶, A, B, G, Q and X are the same as defined above, or a pharmaceutically acceptable salt thereof.

11. The pharmaceutical composition according to claim 10, which is administered simultaneously with application of radiotherapy.

12. The pharmaceutical composition according to claim 10, which is administered before or after application of radiotherapy.

13. A method for treating a patient suffering from malignant tumor comprising administering a therapeutically effective amount of the compound of the formula (I) wherein R¹, R², R³, R⁴, R¹³, R¹⁴, R⁵, R⁶, A, B, G, Q and X are the same as defined above, or a pharmaceutically acceptable salt thereof in combination with another antitumor agent(s) to the patient in need thereof.

14. The method of claim 13, wherein the other antitumor agent is selected from the group consisting of platinum compounds, topoisomerase acting agents, microtubule acting agents and antitumor antibiotics.

15. The method of claim 13 or 14, wherein a therapeutically effective amount of a compound of the formula (I) wherein R¹, R², R³, R⁴, R¹³, R¹⁴, R⁵, R⁶, A, B, G, Q and X are as defined above, or a pharmaceutically acceptable salt thereof is administered simultaneuosly with another antitumor agent.

16. The method of claim 13 or 14, wherein a therapeutically effective amount of a compound of the formula (I) wherein R¹, R², R³, R⁴, R¹³, R¹⁴, R⁵, R⁶, A, B, G, Q and X are as defined above, or a pharmaceutically acceptable salt thereof is administered in combination with another antitumor agent sequentially.

17. A method for treating malignant tumor, comprising administering to a patient undergoing radiotherapy for malignant tumor an effective amount of a compound of the formula (I) wherein R¹, R², R³, R⁴, R¹³, R¹⁴, R⁵, R⁶, A, B, G, Q and X are as defined above, or a pharmaceutically acceptable salt thereof.

18. The method of claim 17, wherein the administration of a compound of the formula (I) wherein R¹, R², R³, R⁴, R¹³, R¹⁴, R⁵, R⁶, A, B, G, Q and X are as defined above, or a pharmaceutically acceptable salt thereof is conducted simultaneously with radiotherapy.

19. The method of claim 17, wherein the administration of a compound of the formula (I) wherein R¹, R², R³, R⁴, R¹³, R¹⁴, R⁵, R⁶, A, B, G, Q and X are as defined above, or a pharmaceutically acceptable salt thereof is conducted before or after radiotherapy.

20. Use of a compound of the formula (I) wherein R¹, R², R³, R⁴, R¹³, R¹⁴, R⁵, R⁶, A, B, G, Q and X are as defined above, or a pharmaceutically acceptable salt thereof for manufacturing a medicament for treating malignant tumor which is administered in combination with another antitumor agent.

21. Use of a combination of a compound of the formula (I) wherein R¹, R², R³, R⁴, R¹³, R¹⁴, R⁵, R⁶, A, B, G, Q and X are as defined above, or a pharmaceutically acceptable salt thereof and another antitumor agent for manufacturing a medicament for treating malignant tumor.

22. Use of claim 20 or 21, wherein the other antitumor agent is selected from the group consisting of platinum compounds, topoisomerase acting agents, microtubule acting agents and antitumor antibiotics.

23. Use of a compound of the formula (I) wherein R¹, R², R³, R⁴, R¹³, R¹⁴, R⁵, R⁶, A, B, G, Q and X are as defined above, or a pharmaceutically acceptable salt thereof for manufacturing a medicament for treating malignant tumor which is administered in combination with radiotherapy for malignant tumor.
